(19) Europäisches Patentamt
European Patent Office
Office européen des brevets

(11) **EP 3 074 060 B1**

(12) **EUROPÄISCHE PATENTSCHRIFT**

(45) Veröffentlichungstag und Bekanntmachung des Hinweises auf die Patenterteilung:
**24.02.2021 Patentblatt 2021/08**

(21) Anmeldenummer: **14812145.2**

(22) Anmeldetag: **21.11.2014**

(51) Int Cl.:
*A61M 1/36* (2006.01)    *A61B 8/00* (2006.01)
*A61B 5/02* (2006.01)    *G01N 29/02* (2006.01)
*G01N 33/49* (2006.01)

(86) Internationale Anmeldenummer:
**PCT/EP2014/075304**

(87) Internationale Veröffentlichungsnummer:
**WO 2015/078786 (04.06.2015 Gazette 2015/22)**

(54) **VERFAHREN ZUR ERMITTLUNG UND/ODER ÜBERWACHUNG DES ZUSTANDS EINES EXTRAKORPORALEN FLUIDES ODER FLUIDSTROMS MITTELS ULTRASCHALL**

METHOD FOR ESTABLISHING AND/OR MONITORING THE STATE OF AN EXTRACORPOREAL FLUID OR FLUID FLOW BY MEANS OF ULTRASOUND

PROCÉDÉ PERMETTANT DE DÉTECTER ET/OU DE SURVEILLER PAR ULTRASONS L'ÉTAT D'UN FLUIDE EXTRACORPOREL OU D'UN ÉCOULEMENT DE FLUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.11.2013 DE 102013224389**

(43) Veröffentlichungstag der Anmeldung:
**05.10.2016 Patentblatt 2016/40**

(73) Patentinhaber: **GAMPT mbH, Gesellschaft für Angewandte**
**Medizinische Physik und Technik**
**06217 Merseburg (DE)**

(72) Erfinder:
• **SCHULTZ, Michael**
**06231 Bad Dürrenberg (DE)**
• **KLAUA, Robert**
**06110 Halle (Saale) (DE)**
• **DIETRICH, Georg**
**06179 Salzmünde OT Zappendorf (DE)**
• **SCHLESINGER, Anton**
**06618 Naumburg (DE)**

(74) Vertreter: **Maikowski & Ninnemann**
**Patentanwälte Partnerschaft mbB**
**Postfach 15 09 20**
**10671 Berlin (DE)**

(56) Entgegenhaltungen:
DE-A1-102005 025 515    DE-A1-102010 034 553
DE-B1- 2 911 258    US-A1- 2004 065 143
US-B1- 6 200 532

**Beschreibung**

Die Erfindung betrifft ein Verfahren zur Ermittlung und/oder Überwachung des Zustands eines extrakorporalen Fluids oder Fluidstroms, insbesondere von Blut oder eines Blutstroms, wobei das Fluid mittels Ultraschall überwacht wird. Das erfindungsgemäße Verfahren ist dadurch gekennzeichnet, dass die mittels der Ultraschallüberwachung ermittelten Merkmale des Fluidzustands mit einer multikriteriellen Ultraschallanalyse verarbeitet werden. Die Erfindung betrifft weiterhin eine Vorrichtung zur Durchführung dieses Verfahrens und die Verwendung dieser Vorrichtung.

Es werden verschiedene extrakorporale Verfahren unterschieden. Am gebräuchlichsten ist die Hämodialyse, darüber hinaus werden die Hämofiltration und Hämodiafiltration angewendet. Weitere extrakorporale Verfahren sind auch die Hämoperfusion, die bei bestimmten akuten Vergiftungen angewendet wird, und das Aphereseverfahren. Extrakorporale Blutkreisläufe finden zudem in Herz-Lungen-Maschinen Anwendung.

Unter Hämodialyse versteht man die Entfernung von Flüssigkeit und gelösten Molekülen aus dem extrakorporal zirkulierenden Blut über Filtersysteme, die in der Regel eine semipermeable Membran enthalten. Die Hämodialyse ist ein sogenanntes Nierenersatzverfahren. Die Dialyse ist neben der Nierentransplantation die wichtigste Nierenersatztherapie bei chronischem Nierenversagen und eine der Behandlungsmöglichkeiten bei akutem Nierenversagen.

Bei der therapeutischen Apherese, die auch als Blutwäsche oder Blutreinigungsverfahren bezeichnet wird, handelt es sich um eine Methode zur extrakorporalen Entfernung von pathogenen Bestandteilen, wie Proteinen, proteingebundenen Substanzen und Zellen, aus dem Blut oder Blutplasma von Patienten. Nach der Entfernung der pathogenen Substanzen wird das gereinigte Blut wieder zurückgeführt. Die Apherese kann auch dazu eingesetzt werden, um Blutbestandteile von einem Menschen zu gewinnen, beispielsweise für die Verwendung als Spendersubstanzen. Aphereseverfahren dienen insbesondere dazu, von einzelnen Spendern ausreichende Mengen von solchen Blutbestandteilen zu gewinnen, die nur einen geringen Anteil des Blutes ausmachen, wie zum Bespiel Thrombozyten oder Blutstammzellen. Bei Aphereseverfahren wird Blut des Spenders aus der Armvene entnommen und in ein geschlossenes, steriles und nur einmal verwendetes Schlauchsystem geleitet. Dort wird es mit einer notwendigen Menge an Antikoagulantia vermischt, die die Gerinnung des Blutes im Apherese-System verhindern sollen. Diese Mischung wird in eine Zentrifuge geleitet, in der die Blutbestandteile entsprechend ihrer Dichte in Schichten aufgetrennt werden. Die zu gewinnenden Blutbestandteile können nun abgetrennt werden. Alle nicht benötigten Blutbestandteile werden dem Blutspender wieder zurückgegeben.

Die Apherese wird beispielsweise in der modernen Krebstherapie oder zur Behandlung von verschiedenen Blutkrankheiten eingesetzt, so zum Bespiel bei der Krankheit *Polycythaemia Vera.*

Die Herz-Lungen-Maschine ist ein medizintechnisches Gerät, das die Pumpfunktion des Herzens sowie die Lungenfunktion für einen beschränkten Zeitraum ersetzen kann. Das Blut wird dabei einer extrakorporalen Zirkulation unterworfen, in dem es dem Körper über ein Schlauchsystem entnommen wird, mit Sauerstoff angereichert und wieder zurückgeführt wird. Eine der häufigsten Anwendungen findet die Herz-Lungen-Maschine in der Herzchirurgie, in der Notfall- und Intensivmedizin werden kleinere Systeme (so genannte Extrakorporale Membranoxygenierung, ECMO) eingesetzt. Bei der Anwendung der Herz-Lungen-Maschine sind Mirkoembolien als Problem bekannt. Die Ursachen der Mirkoembolien können Fibringerinnsel sein, oder auch Plastikpartikel, die von Schlauchoberflächen abgerieben werden oder z.B. aus dem Oxygenator der Herz-Lungen-Maschine stammen.

Obwohl bei der Durchführung des Hämodialyseverfahrens das Blut mit Antikoagulantien, wie z.B. Heparin als häufigstes eingesetztes Antikoagulanz, versetzt wird, besteht die Gefahr des Auftretens von Blutgerinnseln. Dafür gibt es verschiedene Ursachen. Neben falscher Dosierung von Antikoagulantien kann es zum kurzzeitigen Blutstillstand im extrakorporalen Kreislauf kommen. Auf Grund der konstruktiven Vorgaben der verwendeten medizintechnischen Geräte können unter Umständen Stagnationen auftreten. Außerdem kann der Kontakt mit Luft oder mit den künstlichen Oberflächen des extrakorporalen Blutkreislaufes die Blutgerinnung auslösen.

Die Gerinnungsfähigkeit des Blutes ist notwendig, um eine Blutung, beispielsweise nach einer Verletzung, zum Stillstand zu bringen. Sie ist damit eine der wichtigsten Eigenschaften des Blutes. Bei der Blutgerinnung spielen die Thrombozyten (Blutblättchen) eine außerordentlich wichtige Rolle. Die Blutgerinnung folgt der sogenannten Gerinnungskaskade. Nach deren Aktivierung beginnen die Thrombozyten und weitere im Blut enthaltene Gerinnungseiweiße, zu aggregieren und einen Thrombus (auch Gerinnsel genannt) zu bilden. Die Geschwindigkeit der Gerinnselbildung ist abhängig von der Menge der aktiven Gerinnungseiweiße.

Die natürliche und lebensnotwendige Blutgerinnung kann in bestimmten Fällen schaden, nämlich dann, wenn Blut im Gefäßsystem gerinnt. Genauso problematisch ist es, wenn es im extrakorporalen Kreislauf, beispielsweise einer Hämodialysemaschine, einer Herz-Lungen-Maschine oder einer Apherese-Apparatur zur Blutgerinnung kommt. Dies kann dazu führen, dass sich in dem extrakorporalen Kreislauf ein Blutpfropf (Thrombus) bildet. Der Thrombus kann feine Kapillaren in dem extrakorporalen Kreislauf verschließen. Es besteht aber auch die Gefahr, dass ein in dem extrakorporalen Kreislauf gebildeter Thrombus in den Blutkreislauf des Patienten gelangt und dort ein Blutgefäß so dicht verschließt, dass in das nachfolgende Versorgungsgebiet kein Blut mehr fließt und damit die Versorgung mit Sauerstoff und Nährstoffen unterbleibt (Thrombose). Als Folge kann es zum Untergang von Gewebe und sogar zum teilweisen Ausfall bestimmter Organe kommen.

Im extrakorporalen Blutkreislauf, wie z.B. einer Hämodialysemaschine, einer Herz-Lungen-Maschine oder eines Apherese-Apparates ist es deshalb zwingend notwendig, den Gerinnungszustand des Bluts während der Behandlung, und Änderungen des Gerinnungszustands, die während der Behandlung auftreten können oder deren Auftreten durch die Behandlung ausgelöst werden kann, zu überwachen. Hierzu gehört insbesondere die Überwachung von Änderungen der Verteilung von Bestandteilen des Bluts, insbesondere der Blutzellen, und von Änderungen der Viskosität des Bluts. Damit soll eine unerwünschte beginnende Blutgerinnung und damit im Zusammenhang die mögliche Bildung von Blutgerinnseln frühzeitig erkannt werden. Änderungen des Gerinnungszustands des Bluts, Blutgerinnsel und andere gefährliche Bestandteile sollten in dem extrakorporalen Kreislauf mit geeigneten Maßnahmen korrigiert bzw. zurückgehalten werden, so dass negative Auswirkungen auf den zu behandelnden Patienten vermieden werden können.

Herkömmliche Behandlungssysteme umfassen deshalb Mittel zur Erkennung von gefährlichen Bestandteilen im extrakorporalen Blutkreislauf, wie z.B. Luftblasen, und verfügen weiterhin über entsprechende Einrichtungen, die einen Alarm auslösen und/oder zu einem Behandlungsstopp führen. Blutgerinnsel versucht man mittels sogenannter Gerinnselfänger zurückzuhalten, bevor sie in den Körper eines Patienten gelangen können.

Aus dem Stand der Technik sind bereits Verfahren und Vorrichtungen zur Überwachung extrakorporaler Kreisläufe auf darin enthaltene gefährliche Bestandteile bekannt.

Die DE 10 2010 034 553 offenbart eine Vorrichtung zur Ermittlung und/oder Überwachung von Fremdstrukturen in einem Fluid oder einem Fluidstrom sowie ein diesbezügliches Verfahren.

Dieses System ist insbesondere in der Lage, in dem Fluidstrom mittels einem Ultraschallüberwachungsmittel Luftblasen zu detektieren. Jedoch können Blutgerinnsel nur mit einem zusätzlichen optischen Überwachungsmittel nicht aber mittels Ultraschall im Blut ermittelt werden.

DE 2911258 B1 offenbart eine Vorrichtung zum nichtinvasiven Messen der Blutströmgeschwindigkeit nach der Ultraschall-Doppler-Effekt-Methode, mit der die Blutströmgeschwindigkeit im Bereich der kleinen und kleinsten Gefäße (Mikrozirkulation) gemessen und/oder eine Erythrozyten-Aggregation erfasst werden kann.

US 5928180 offenbart ein Verfahren und eine Vorrichtung für die Echtzeit-Überwachung des Blutvolumens in einem Blutfilter, die es ermöglichen, den Filter und die Leistung einer Dialysemaschine zu kontrollieren und frühzeitig vor einer Gerinnung zu warnen.

DE 10311408 B3 offenbart ein Verfahren zur nichtinvasiven Messung der Konzentration von Blutbestandteilen in zentralen Blutgefäßen, insbesondere der Hämoglobinkonzentration oder der Sauerstoffsättigung des Blutes durch das Messen von rückgestreutem Licht unter Einwirkung einer Ultraschallstrahlung.

Aus US2004065143 ist ein Verfahren zur Messung u.a. der Dichte und Viskosität von Blutproben mittels Ultraschall bekannt. Mit den im Stand der Technik offenbarten Verfahren und Vorrichtungen ist es bislang aber nicht möglich, Änderungen des Gerinnungszustands, insbesondere das Einsetzen einer unerwünschten Blutgerinnung, instantan zuverlässig nachzuweisen. Die auf der Streuwirkung des Ultraschallsignals an den Blutgerinnseln basierenden Verfahren und Vorrichtungen sind nicht zuverlässig, da die Streuwirkung der Blutgerinnsel in der Regel zu gering ist.

Es ist daher die Aufgabe der vorliegenden Erfindung, ein Verfahren und eine Vorrichtung bereitzustellen, die es ermöglichen, Änderungen des Zustands von Blut, insbesondere des Gerinnungszustands schnell, sicher und mit einer möglichst einfachen Messanordnung zu erkennen. Aufgabe der Erfindung ist insbesondere der Nachweis vom Normalzustand abweichender Eigenschaften des menschlichen Blutes und die Überwachung des zeitlichen Verlaufs von Veränderungen des Blutzustands, die zu Abweichungen vom Normalzustand führen.

In Tabelle 1 sind die Durchschnittswerte beispielhafter physikalischen Bluteigenschaften für einen gesunden erwachsenen Menschen aufgelistet.

**Tabelle 1:** Durchschnittliche Eigenschaften von menschlichem Blut

| | |
|---|---|
| Farbe | rot |
| Aggregatzustand | flüssig |
| betrachteter Temperaturbereich | 35,8 °C bis 37,2 °C |
| Dichte | 1,057 g/ml |
| Viskosität bei 37 °C | 4 mPas bis 25 mPas |
| Schallgeschwindigkeit | 1483 m/s |
| Ø Erythrozyten | 6 $\mu$m bis 15 $\mu$m |
| Hämatokrit | 37 % bis 50 % |

Das erfindungsgemäße Verfahren und die erfindungsgemäße Vorrichtung sollen es ermöglichen, die das Auftreten von unerwünschten Veränderungen des Blutzustands, insbesondere einer unerwünschten Blutgerinnung instantan in einem extrakorporalen Kreislauf möglichst frühzeitig und mit hoher Zuverlässigkeit nachzuweisen.

Diese Aufgabe wird gelöst durch ein Verfahren mit den Merkmalen des Anspruchs 1. Es wird ein Verfahren zur Ermittlung und/oder Überwachung des Zustandes eines Fluides oder eines Fluidstroms bereitgestellt, wobei das Fluid mittels Ultraschall überwacht wird. Die mittels der Ultraschallüberwachung ermittelten Merkmale des Fluidzustands werden anschließend mit einer multikriteriellen Ultraschallanalyse, insbesondere einem Auswertealgorithmus, verarbeitet, wodurch eine Änderung der Verteilung von in dem Fluid enthaltenen Teilchen, und/oder Änderungen der Viskosität des Fluides gemessen werden und der Fluidzustand zuvor definierten Zuständen zugewiesen wird.

Bei dem Fluid bzw. Fluidstrom handelt es sich vorzugsweise um eine Flüssigkeit, die sowohl gelöste Stoffe als auch suspendierte Partikel enthalten kann. In einer bevorzugten Ausführungsform der Erfindung ist das Fluid eine Suspension. Eine Suspension ist ein heterogenes Stoffgemisch aus einer Flüssigkeit und darin fein verteilten Festkörpern, die in der Flüssigkeit mit geeigneten Aggregaten (Rührer, Dissolver, Flüssigkeitsstrahlen, Nassmühle) sowie meist mithilfe zusätzlicher Dispergiermittel aufgeschlämmt und in der Schwebe gehalten werden. Eine Suspension ist eine grobdisperse Dispersion und tendiert zur Sedimentation und Phasentrennung. Die Feststoffe sind in der flüssigen Phase suspergiert. Der Fluidzustand ist insbesondere der Misch- bzw. Entmischungszustand der einzelnen Phasen.

In einer weiteren bevorzugten Ausführungsform der Erfindung ist das Fluid eine Dispersion. Eine Dispersion ist ein heterogenes Gemisch aus mindestens zwei Stoffen, die sich nicht oder kaum ineinander lösen oder chemisch miteinander verbinden. Dabei ist ein Stoff (disperse Phase) fein verteilt in einem anderen Stoff (Dispersionsmedium). In der Regel handelt es sich um Kolloide. Die einzelnen Phasen können deutlich voneinander abgegrenzt und in der Regel durch physikalische Methoden wieder voneinander getrennt werden (z. B. Filtrieren, Zentrifugieren), oder sie entmischen sich von selbst (Sedimentieren). Der Fluidzustand ist insbesondere der Misch- bzw. Entmischungszustand der einzelnen Phasen.

In einer weiteren bevorzugten Ausführungsform der Erfindung ist das Fluid eine Emulsion. Unter einer Emulsion versteht man ein fein verteiltes Gemisch zweier normalerweise nicht mischbarer Flüssigkeiten ohne sichtbare Entmischung. Beispiele für Emulsionen sind zahlreiche Kosmetika, Milch oder Mayonnaise. Der Fluidzustand ist insbesondere der Misch- bzw. Entmischungszustand der einzelnen Flüssigkeiten.

Gemäß der Erfindung ist das Fluid Blut, und zwar Blut in einem extrakorporalen Kreislauf. Der Fluidzustand ist insbesondere der Gerinnungszustand von Blut.

In einer bevorzugten Ausführungsform wird ein Verfahren zur Ermittlung und/oder Überwachung des Zustandes von Blut oder eines Blutstroms, insbesondere eines extrakorporalen Blutstroms, bereitgestellt, wobei das Blut mittels Ultraschall überwacht wird. Die mittels der Ultraschallüberwachung ermittelten Merkmale des Blutzustandes werden mit einer multikriteriellen Ultraschallanalyse, insbesondere einem Auswertealgorithmus, verarbeitet, wodurch eine Änderung der Verteilung von Bestandteilen im Blut und/oder Änderungen der Viskosität des Bluts gemessen werden und der Blutzustand zuvor definierten Zuständen, insbesondere Gerinnungszuständen, zugewiesen wird.

Das erfindungsgemäße Verfahren hat den Vorteil, dass Änderungen des Zustands eines Fluides, insbesondere des Gerinnungszustands von Blut frühzeitig, vorzugsweise vom Beginn des Eintretens der Zustandsänderung an, festgestellt werden können.

Durch das erfindungsgemäße Verfahren ist es auch möglich, wenigstens eine Fremdstruktur im Fluid zu erkennen bzw. von wenigstens einer zweiten Fremdstruktur zu unterscheiden.

Die wenigstens eine erste Fremdstruktur ist insbesondere ein Festkörper, vorzugsweise ein Blutgerinnsel.

Die wenigstens eine zweite Fremdstruktur kann ein weiterer Festkörper, wie beispielsweise ein von einer Oberfläche des extrakorporalen Kreislaufs abgelöster Fremdkörper, wie zum Beispiel Abrieb von dem Fluidführungsmittel, sein. Die eine zweite Fremdstruktur kann aber beispielsweise auch eine Luftblase ein.

Vorzugsweise ist mit dem erfindungsgemäßen Verfahren die Unterscheidung zwischen Blutgerinnseln und Fremdkörpern anderer Art, wie Luft, möglich. Die Unterscheidung kann über die Rückstreuamplitude erfolgen, die beispielsweise bei Luftblasen größer als bei Blutgerinnseln ist, da der Impedanzsprung zwischen Blut und Luft erhöht ist.

Zur Ultraschallüberwachung kann jede herkömmliche bekannte Messmethode herangezogen werden. Vorzugsweise erfolgt die Ultraschallüberwachung mittels Messung der Rückstreuung, Frequenzverschiebung (Doppler) und/oder Viskosität (Elastographie).

Das Verfahren beruht insbesondere auf der Erhöhung des Ultraschall-Rückstreusignals bei Auftreten von Blutgerinnseln. Die Blutgerinnung (Hämostase) ist ein komplexer biochemischer Vorgang, dessen Ablauf, genauso wie die daran beteiligten Stoffe, noch nicht vollständig erforscht ist. Bei Auslösung der Gerinnung werden ein oder mehrere Gerinnungsfaktoren aktiviert, die ihrerseits kaskadenartig weitere Faktoren aktivieren.

Blut ist, stoffkundlich gesehen, eine Suspension von Blutzellen in Blutplasma. Dabei sind hauptsächlich drei verschiedene Zellarten zu unterscheiden: rote (Erythrozyten) und weiße (Leukozyten) Blutkörperchen sowie Blutplättchen (Thrombozyten). Wie in Tabelle 2 abzulesen, ist der Streuquerschnitt, definiert als Produkt der Fläche mit der Anzahl für rote Blutkörperchen, viel größer als der jeder anderen Zellart.

**Tabelle 2:** Vergleich der Blutzellen

| Typ | Durchmesser d [$\mu$m] | Anzahl N pro $\mu$l Blut | Gesamtfläche $\frac{\pi}{4}d^2N$ [mm$^2$] |
|---|---|---|---|
| Erythrozyten | 7,5 | $4 \times 10^6$ bis $5 \times 10^6$ | $1,3 \times 10^3$ bis $1,7 \times 10^3$ |
| Leukozyten | 7 bis 20 | $4 \times 10^3$ bis $9 \times 10^3$ | 0,2 bis 2,8 |
| Thrombozyten | 1,5 bis 3 | $1,5 \times 10^5$ bis $3 \times 10^5$ | 0,3 bis 2,1 |

Da sich zudem die festen Bestandteile des Blutes in ihren akustischen Eigenschaften kaum vom Blutplasma unterscheiden und insgesamt nur sehr wenige Streuprozesse auftreten, werden für Rückstreuprozesse im Blut üblicherweise nur Rückstreuungen an Erythrozyten berücksichtigt. Des Weiteren wird angenommen, dass rote Blutkörperchen kugelförmige Streukörper sind, die nicht miteinander wechselwirken. Beide Annahmen gelten für die üblicherweise verwendeten Blutersatzflüssigkeiten uneingeschränkt, für Humanblut jedoch nur teilweise: die bikonkaven Scheiben sind in turbulenter Strömung willkürlich ausgerichtet und ändern ihre Ausrichtung ständig. Daher kann davon ausgegangen werden, dass in einem bestimmten Raumwinkel die sichtbare Fläche immer gleich groß, der Streuquerschnitt also richtungsunabhängig ist. Betrachtet man jedoch eine laminare Strömung, wie sie in den großen Adern und in künstlichen Blutgefäßen vorliegt, so richten sich die Erythrozyten in der Strömung aus, um ihren Strömungswiderstand zu verringern. Aufgrund des Volumenanteils von Blutzellen am Gesamtblutvolumen von bis zu 50 %, wovon der Großteil Erythrozyten sind, stoßen die Erythrozyten ständig aneinander. Indem sie den Abstand zu benachbarten Zellen minimieren, verringert sich ihr Strömungswiderstand erheblich. Diese Erythrozytenaggregation, aufgrund ihrer charakteristischen Form auch Geldrollenbildung genannt, führt zum einen zu einer Richtungsabhängigkeit des Streusignals und zum anderen zu einer Signalerhöhung.

Nicht zu verwechseln ist die Erythrozytenaggregation mit der Thrombozytenaggregation, welche eine Vorstufe der Gerinnung darstellt. Aufgrund der viel kleineren Abmessungen ist eine Änderung des Ultraschall-Rückstreusignals allein auf Basis der der Thrombozyten nicht sichtbar.

Vereinfacht beruht die Gerinnung auf der Verbindung der roten Blutkörperchen mit Fibrinfäden zu einem unauflöslichen Netz, das z. B. eine Wunde verschließen oder aber ein Blutgefäße verstopfen kann. Es handelt sich bei der Gerinnung also um einen Effekt, der sowohl positive als auch negative Folgen haben kann.

Der Erfindung liegt der überraschende Befund zugrunde, dass sich der Gerinnungszustand als Eigenschaft des Blutes dann sicher über Ultraschallmessungen detektieren lässt, wenn nicht nur Änderungen der Verteilung der Blutbestandteile mittels Rückstreuung, sondern zusätzlich und/oder gleichzeitig auch Änderungen der Viskosität des Bluts und/oder Blutstroms gemessen werden. Die Vernetzung der Blutkörperchen mit Fibrinfäden bei der Blutgerinnung führt nicht nur zu signifikanten Erhöhung des Streuquerschnitts der Blutbestandteile, die über die Ultraschallrückstreuung messbar sind, sondern auch zu einer Viskositätsänderung, die mittels Elastographiemessungen detektierbar sind.

In einer besonders bevorzugten Ausführungsform erfolgt die Ultraschallüberwachung des Fluidstroms dadurch, dass der Fluidstrom zeitharmonisch mechanisch und/oder transient mechanisch angeregt und das Ultraschall-Rückstreusignal hinsichtlich der Dopplerfrequenzverschiebung sowie der Elastographie analysiert wird. Hieraus kann auf Veränderungen der dynamischen Viskosität von Blut sowie Änderungen der Verteilung der Blutzellen geschlossen werden, die sich hervorragend als Marker für Gerinnungszustände von Blut eignen.

Vorzugsweise werden in dem Fluidstrom niederfrequente harmonische und/oder nicht harmonische Wellen angeregt, die zur Charakterisierung der elastischen Eigenschaften des Fluides oder Fluidstroms herangezogen werden. Die Anregung der Wellen kann mechanisch, pneumatisch und/oder akustisch, wie nachfolgend an Beispielen dargestellt, erfolgen:

mechanisch:

- Anregung durch Schütteln bzw. Klopfen
- periodische Deformation des Schlauches
- Ausnutzung von Rollenpumpenstößen zur Anregung
- Einsatz von Peristaltikpumpen

pneumatisch:

- Anregung mittels pneumatisch gesteuerter Druckmanschetten
- Anregung durch Druckvariation im Reservoir
  (Ausbreitung der Wellen im Medium bis hin zur Messstelle)

akustisch:

- Anregung mit niederfrequenten Schallwandlern (Lautsprecher)
- Ankopplung über Luft oder geeignete Schallvorlaufstrecken
- Anregung mit Ultraschallburst mit geeigneter PRF (pulse repetition frequency)

Das erfindungsgemäße Verfahren dadurch gekennzeichnet, dass mittels der Ultraschallüberwachung eine Vielzahl von Merkmalen des Blutzustandes instantan, d. h. augenblicklich, ermittelbar sind. Mit einer "Vielzahl von Merkmalen des Blutzustandes" sind die physikalischen Parameter gemeint, die mittels Ultraschallanalyse, vorzugsweise Ultraschall-Rückstreuung, messbar sind, und die durch die fortschreitende Blutgerinnung verändert werden können. Diese sind vorzugsweise ausgewählt aus der Schallgeschwindigkeit, der Viskosität, dem Durchmesser der Streukörper, der Standardabweichung der Rückstreuung, der maximalen Abweichung der Rückstreuung, dem Maß der Regelmäßigkeit der Anordnung der Streukörper, der Turbulenz und der Geschwindigkeitsverteilung der Streukörper. Das erfindungsgemäße Verfahren hat unter anderem den Vorteil, dass nicht nur die fortschreitende Blutgerinnung, sondern auch das Einsetzen bzw. der Beginn der Blutgerinnung instantan detektiert werden kann. Besonders bevorzugt für die Detektion der beginnenden Blutgerinnung sind die Parameter Viskosität und Streukörperdurchmesser, die Standardabweichung der Rückstreuung, die maximale Abweichung der Rückstreuung, das Maß der Regelmäßigkeit der Anordnung der Streukörper, die Turbulenz und die Geschwindigkeitsverteilung der Streukörper. Der Streukörperdurchmesser kann sich signifikant von etwa 7 $\mu$m eines durchschnittlichen Erythrozyten auf mehrere 10 bis 100 $\mu$m eines Mikrogerinnsels ändern. Vorzugsweise können mit dem erfindungsgemäßen Verfahren Streukörperdurchmesser, beispielsweise Durchmesser von Blutgerinnseln im Bereich von 10 bis 150 $\mu$m, mehr bevorzugt im Bereich von 10 bis 100 $\mu$m, ebenso bevorzugt im Bereich von 15 bis 80 $\mu$m, besonders bevorzugt im Bereich von 15 bis 50 $\mu$m detektiert werden.

Wie oben beschrieben, beruht das erfindungsgemäße Verfahren darauf, dass die mittels der Ultraschallüberwachung ermittelten Merkmale des Fluidzustandes, insbesondere des Blutzustandes, mittels einer multikriterillen Ultraschallanalyse weiterverarbeitet werden. In einer bevorzugten Ausführungsform ist das erfindungsgemäße Verfahren dadurch gekennzeichnet, dass die multikriterielle Ultraschallanalyse des Fluidzustands mittels eines nachfolgend beschriebenen Auswertealgorithmus erfolgt.

Der Überwachungsalgorithmus des Fluidzustandes besteht aus einem Mehrschichtenmodell.

In Ebene 1 (unterste Schicht) werden die Merkmale der Ultraschallanalyse von Rückstreuung, Doppler-Frequenzverschiebung und/oder Elastographie dargestellt. Ihre Darstellung kann linear und nichtlinear mit Hilfe von Darstellungsparametern verändert werden.

In Ebene 2 wird eine anwendungsbezogene, statistisch, numerische Klassifikation oder Regression auf Basis der Merkmale aus Ebene 1 vorgenommen. Die Klassifikation wird in eine manuelle/maschinelle (beaufsichtigte) oder automatische (unbeaufsichtigte) Klassifikation unterschieden. Ebenso ist die Verknüpfung beider Methoden möglich. Während die Klassifikation Muster in den Merkmalen sucht und zuweist, stellt die Regression den Zusammenhang einer abhängigen Variable (z.B. der Fluidzustand) und einer oder mehreren unabhängigen Variablen dar (z.B. Viskosität und Varianz der Teilchen innerhalb der Suspension) in einem quantitativen Zusammenhang dar. Die Darstellungsparameter der Ebene 1 werden über maschinelles Lernen online oder offline optimiert, um die Merkmale optimal (Minimierung der Fehler) zu ihrer Analyse darzustellen.

In Ebene 3 (oberste Schicht) erfolgt auf Basis der zeitdiskret ermittelten Fluidzustände in Ebene 2 die Formulierung einer Zustandsraumdarstellung. Unter Anwendung eines Bayes'schen Minimum-Varianz-Schätzers für lineare stochastische Systeme (Kalman, R. E., 1960, A new approach to linear filtering and prediction problems. Transaction of the ASME, Journal of Basic Engineering, pp. 35 - 45) wird aus den mit Unsicherheit behafteten Ergebnissen der Ebene 2 in optimaler (Minimierung der Fehler) Art und Weise auf den Systemzustand des Fluids geschlossen. Das Zustandsraummodell schließt dabei alle verfügbaren Informationen ein, d. h. sowohl das Wissen um den physikalischen Prozess des Fluids und die Dynamik des Messsystems, die zugrundeliegenden statistischen Prozesse des Systemrauschens, der Messfehler und die Unsicherheit im physikalischen Modell, sowie die Anfangsbedingungen.

Ziele dieses Verfahrens sind

- eine schnelle und möglichst genaue Zustandsschätzung des Fluids,
- eine hohe Unempfindlichkeit gegenüber Störungen, seien diese im individuellen physiologischen Zustand eines Patienten zu finden oder systembedingt,
- eine schnelle Reaktionszeit auf Veränderungen, unabhängig, ob diese beabsichtigt (z. B. Änderung der Medikation am Patienten) oder unbeabsichtigt sind, z. B. Blutgerinnung,
- eine starke Redundanzreduktion der vorhandenen Merkmale in Ebene 1, hin zu wenigen Merkmalen hoher Unterscheidbarkeit von physikalischen Fluidzuständen,
- die online Überwachung der Dynamik eines Fluids.

In einer besonders bevorzugten Ausführungsform umfasst der Auswertealgorithmus des erfindungsgemäßen Verfahrens die nachfolgenden Signalverarbeitungsstufen:

a. Empfangen eines Ultraschallsignals oder mehrerer Ultraschallsignale,
b. Merkmalsextraktion aus dem (den) Ultraschallsignal(en),
c. Mustererkennung,
d. Nachverarbeitung, und
e. Zuordnung einer Punktzahl, wobei die Punktzahl die Güte (Wahrscheinlichkeit) für ein gegebenes Klassifikationsresultat definiert.

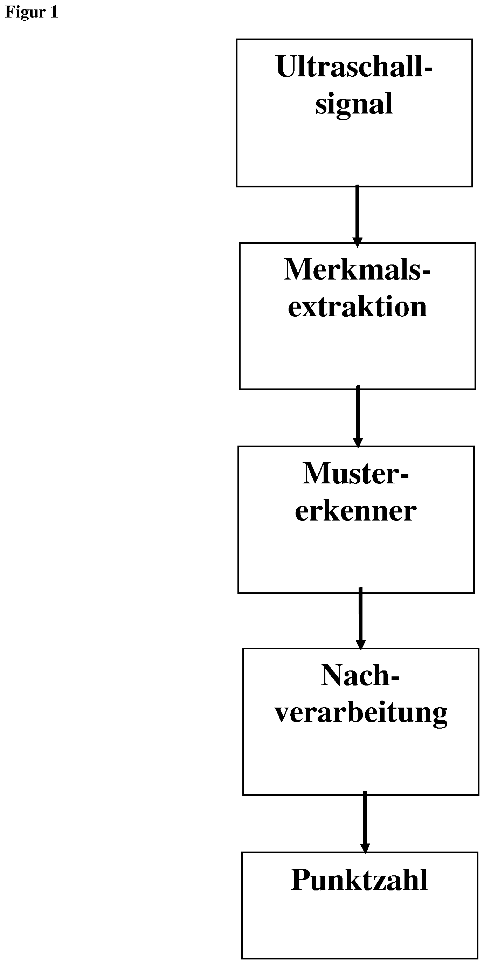

Die vorgestellte Ausführungsform beruht auf dem allgemeinen Aufbau eines Auswertealgorithmus. Abbildung 1 stellt die Verarbeitungsstufen eines geeigneten Auswertealgorithmus dar. Aus den Ultraschallsignalen werden Merkmale entnommen, die möglichst gut Veränderungen der Koagulation beschreiben. Aus diesem Grund wird ein Fluid, vorzugsweise Blut, bei kontrollierten physikalischen Veränderungen wie beispielsweise harmonischen Bewegungsanregungen, untersucht, um die zwischen den in dem Fluid enthaltenen Partikeln, vorzugsweise zwischen den Blutzellen, auftretenden Veränderungen abzubilden. Hieran folgen der Mustererkenner und eine Nachverarbeitung der Resultate, um eine möglichst schlüssige Klassifikationen zu erreichen. Die "Punktzahl" bezeichnet schließlich den wahrscheinlich verbleibenden Fehler in der Beschreibung des Zustandes des Fluids, vorzugsweise des Bluts, und stellt damit die Risikoabschätzung bei abgeleiteten Eingriffen am Patienten dar.

Der Mustererkenner in der vorgestellten Ausführungsform basiert auf einer seriellen Anwendung der Hauptkomponentenanalyse (englisch: principal component analysis, kurz PCA) und der Linearen Diskriminanzanalyse (englisch: linerar discriminant analyses, kurz LDA). Dieser Klassifikationsansatz zeigt sich ebenso leistungsfähig wie die oft verwendete Support Vector Machine. Er eignet sich jedoch besser für Unterscheidungsprobleme mit mehr als 2 Klassen (Li et al., 2003, Using discriminant analysis for multi-class classification. Proceedings of the Third IEEE International Conference on Data Mining, page 589. IEEE Computer Society.). Die PCA ermöglicht zunächst, die in den Merkmalen enthaltene Signalinformation in eine kompakte Repräsentation zu transformieren. Die PCA ist hierfür der optimale Ansatz, jedoch ist sie nicht optimal in Bezug auf die Diskriminanz zwischen den Klassen. Zu diesem Zweck wird nachfolgend die LDA angewandt, um durch eine Koordinatentransformation die Klassen optimal zu trennen.

Das erfindungsgemäße Verfahren, insbesondere der erfindungsgemäße Auswertealgorithmus, ist dazu geeignet, Kriterien der höchsten Diskriminanz bereits in den "Rohmaterialien" aufzuspüren. In einer weiteren Ausführungsform liegt dem erfindungsgemäßen Verfahren, insbesondere dem erfindungsgemäßen Auswertealgorithmus, ein Maschinenlernalgorithmus zu Grunde (eine stochastische Optimierung der Darstellungsparameter der Merkmale wurde mit einem genetischen Algorithmus vorgenommen), bei dem alle Signalverarbeitungsstufen, insbesondere die Merkmalsextraktion, offline optimiert werden.

In einer weiteren bevorzugten Ausführungsform wird der Auswertealgorithmus durch eine fortlaufende Optimierung an das Erkennungsproblem online angepasst. In einer besonders bevorzugten Ausführungsform des erfindungsgemäßen Verfahrens lernt der Auswertealgorithmus zusätzlich den individuellen Zustand des Fluids, insbesondere des Blutzustandes, ebenfalls online. Durch diese Optimierung kann eine weitere Verbesserung der Robustheit des Auswertealgorithmus erzielt werden.

Mit Hilfe des erfindungsgemäßen Verfahrens ist es möglich, das der Auswertealgorithmus online Veränderungen des Zustandes des Fluids, insbesondere des Blutzustandes, detektiert. Dadurch können mit Hilfe des Auswertealgorithmus der Gerinnungszustand von Blut bzw. Veränderungen des Gerinnungszustandes von Blut online und mit hoher Zuverlässigkeit detektiert werden.

Der Vorteil des beschriebenen Auswertealgorithmus bei der Bestimmung von Gerinnung in Blut in Bezug auf bekannte Verfahren des Standes der Technik besteht in der Verknüpfung einer multikriteriellen Merkmalsextraktion aus dem Ultraschall-Rückstreusignal und einer leistungsfähigen Analyse des Fluidzustands. Im Besonderen wird der Fluidstrom, beispielsweise eine fließende Suspension, wie ein Blutstrom, zeitharmonisch mechanisch und/oder transient mechanisch angeregt und das Ultraschall-Rückstreusignal hinsichtlich der Dopplerfrequenzverschiebung sowie der Elastographie analysiert. Hieraus kann auf die dynamische Viskosität von Blut sowie die Veränderung der Verteilung der Blutzellen geschlossen werden, die sich hervorragend als Marker für Gerinnungszustände von Blut eignen. Der Auswertealgorithmus adaptiert sich an die Datenbasis und isoliert Merkmale, welche die höchste Unterscheidbarkeit innerhalb der unterschiedlichen Gerinnungszustände aufweisen. Auf dieses Weise können große Datenmengen an Merkmalen effektiv ausgewertet werden.

Das erfindungsgemäße Verfahren hat weiterhin den Vorteil, dass die online bzw. instantan detektierten Veränderungen des Gerinnungszustandes des Blutes genutzt werden können, um Interventionen vorzunehmen. Solche Interventionen können zum Beispiel das sofortige Stoppen des extrakorporalen Kreislaufes in beispielsweise einer Hämodialysemaschine oder aber das gezielte [Unbekannt1]Zudosieren von Gerinnungshemmern sein. Das erfindungsgemäße Verfahren hat deshalb insbesondere den Vorteil, das auf Veränderungen des Gerinnungszustandes von Blut in einem extrakorporalen Kreislauf innerhalb kürzester Zeit, beispielsweise weniger Sekunden, reagiert werden kann und somit Schaden von dem Patienten sicher abgewendet werden kann.

Das Verfahren gemäß der vorliegenden Erfindung ist auch dazu geeignet, Fehler in einem Fluidführungsmittel einer Vorrichtung zu detektieren. Das Fluidführungsmittel kann beispielsweise ein Schlauchsystem sein. Die Vorrichtung ist vorzugsweise eine Vorrichtung zur Blutbehandlung, wie zum Beispiel eine Hämodialysemaschine, eine Herz-Lungen-Maschine oder eine Apherese-Maschine.

Es ist insbesondere ein Vorteil des erfindungsgemäßen Verfahrens, dass das Verfahren nichtinvasiv ist. Dieses Verfahren hat also den Vorteil, dass auf invasive Untersuchungen, wie zum Beispiel Blutentnahmen, am Patienten verzichtet werden kann. Ein weiterer Vorteil des erfindungsgemäßen Verfahrens besteht darin, dass der Fluidzustand, insbesondere der Blutzustand, fortlaufend und kontinuierlich gemessen werden kann.

Die vorliegende Erfindung betrifft außerdem eine Vorrichtung zur Durchführung des erfindungsgemäßen Verfahrens. Diese Vorrichtung umfasst wenigstens ein Ultraschallüberwachungsmittel und wenigstens ein Signalauswertemittel. Die erfindungsgemäße Vorrichtung ist dadurch gekennzeichnet, dass die mittels der Ultraschallüberwachung ermittelten Merkmale des Fluidzustandes, insbesondere des Blutzustandes, mittels einer multikriteriellen Analyse verarbeitet werden. Für die multikriterielle Ultraschallanalyse wird vorzugsweise der oben beschriebene Auswertealgorithmus (siehe auch Figur 1) in der erfindungsgemäßen Vorrichtung verwendet. Durch die Verarbeitung der mittels der Ultraschallüberwachung ermittelten Merkmale des Fluidzustandes, insbesondere des Blutzustandes, ist es möglich, Änderungen der Verteilung von in dem Fluid enthaltenen Teilchen, insbesondere Blutbestandteilen, und/oder Änderungen der Viskosität des Fluides, insbesondere Bluts, zu messen und den Fluidzustand, insbesondere den Blutzustand, vordefinierten Zuständen zuzuweisen.

Die erfindungsgemäße Vorrichtung hat den Vorteil, dass Änderungen des Zustands eines Fluides, insbesondere des Gerinnungszustands von Blut frühzeitig, vorzugsweise vom Beginn des Eintretens der Zustandsänderung an, festgestellt werden können.

Mit der erfindungsgemäßen Vorrichtung ist es auch möglich, wenigstens eine Fremdstruktur im Fluid zu erkennen bzw. von wenigstens einer zweiten Fremdstruktur zu unterscheiden. Bei den zuvor definierten Zuständen handelt es sich bei Blut insbesondere um Gerinnungszustände. Bei der Erkennung von Festkörpern im Blut geht es vor allem darum, Blutgerinnsel zu erkennen.

Grundsätzlich können bei der Ultraschallüberwachung alle Messprinzipien eingesetzt werden, durch die Fremdstrukturen, wie zum Beispiel Festkörper, insbesondere Blutgerinnsel detektiert werden können. Bevorzugte Ultraschallüberwachungsmittel gemäß der vorliegenden Erfindung beruhen auf der Messung der Rückstreuung, Frequenzverschiebung (Doppler) und/oder Viskosität (Elastographie). In einer besonders bevorzugten Ausführungsform kommt in der erfindungsgemäßen Vorrichtung ein Ultraschallüberwachungsmittel zum Einsatz, das auf der Messung der Rückstreuung beruht.

Das in der erfindungsgemäßen Vorrichtung integrierte Ultraschallüberwachungsmittel kann zum Beispiel ein auf Ultraschall-Rückstreuung basierendes Messverfahren zur Partikelcharakterisierung nutzen. Kerngedanke des Aufbaus eines solchen Ultraschallüberwachungsmittels ist der Einsatz breitbandiger Sende-/Empfangs-Wandler, die hinsichtlich einer notwendigen Sendeleistung und eines dem Durchmesser des Fluidführungsmittels entsprechenden Messfensters optimal aufeinander abgestimmt sind. Auf dieser Weise ist der von den Partikeln gestreute Schallanteil erfassbar und hinsichtlich der Intensität, der Laufzeit (entspricht der Eindringtiefe) und der Schallfrequenz analysierbar. Bei der Anordnung der Ultraschall-Wandler bestehen verschiedene Möglichkeiten. Zum einen kann die direkte Rückstreuung mittels eines einzigen Sende-/Empfangs-Wandlers mit Vorlaufstrecke gemessen werden. Hierbei erfolgt eine klassische Reflexionsmessung. Zum anderen können auch die unter definierten Winkeln gestreuten Schallanteile analysiert werden. Dazu wird in der Regel ein getrenntes Ultraschall-Wandlerpaar eingesetzt.

Es ist aber auch denkbar, dass das in der erfindungsgemäßen Vorrichtung integrierte Ultraschallüberwachungsmittel zum nichtinvasiven Messen von Fremdbestandteilen in dem Fluidstrom auf der Ultraschall-Doppler-Effekt-Methode unter Verwendung von Ultraschall-Sendern/Empfängern für den am strömenden Blut reflektierten Ultraschall und unter Verwendung von Dopplerfrequenz-Verschiebung zwischen Sende- und Empfangsfrequenz ermittelnden Signalauswertemitteln beruht.

Es ist ferner auch denkbar, dass das in der erfindungsgemäßen Vorrichtung integrierte Ultraschallüberwachungsmittel zum nichtinvasiven Messen von Fremdbestandteilen in dem Fluidstrom auf der Elastographie-Methode zur Bestimmung der dynamischen Viskosität unter Verwendung von Ultraschall-Sendern/Empfängern und einer Vorrichtung zur zeitharmonischen mechanischen und/oder transienten mechanischen Anregung des Fluides beruht.

In einer besonders bevorzugten Ausführungsform beruht das in der erfindungsgemäßen Vorrichtung integrierte Ultraschallüberwachungsmittel zum nichtinvasiven Messen von Fremdbestandteilen in dem Fluidstrom auf der Elastographie-Methode zur Bestimmung der dynamischen Viskosität unter Verwendung von Ultraschall-Sendern/Empfängern und einer Vorrichtung zur zeitharmonischen mechanischen und/oder transienten mechanischen Anregung des Fluides und zusätzlich auf der Ultraschall-Doppler-Effekt-Methode unter Verwendung von Ultraschall-Sendern/Empfängern für den am strömenden Blut reflektierten Ultraschall und unter Verwendung von Dopplerfrequenz-Verschiebung zwischen Sende- und Empfangsfrequenz ermittelnden Signalauswertemitteln.

Das Signalauswertemittel umfasst vorzugsweise einen konventionellen Prozessor, eine Software zur Steuerung der Komponenten und zur Signalverarbeitung und -umwandlung. Bei dem Signalauswertemittel handelt es sich beispielsweise um einen Steuercomputer oder um einen Prozessrechner, wobei der Steuercomputer oder Prozessrechner auch die mit der erfindungsgemäßen Vorrichtung in Verbindung stehende Steuereinheit einer Blutbehandlungsvorrichtung, wie beispielsweise einer Hämodialysemaschine, Herz-Lungen-Maschine oder Apherese-Apparatur sein kann.

Es ist insbesondere vorgesehen, dass mit dem Signalauswertemittel ein oder mehrere Festkörper im Fluid, insbesondere Blutgerinnsel, in dem extrakorporalen Kreislauf detektierbar sind und detektiert werden.

In einer weiteren bevorzugten Ausführungsform wird die Detektion wenigstens einer Fremdstruktur in dem Fluid oder Fluidstrom von dem Signalauswertemittel als ein signalauslösendes Ereignis registriert und das Signalauswertemittel kann daraufhin ein Signal abgeben. Vorzugsweise wird durch das Signalauswertemittel dann ein Signal abgegeben, wenn wenigstens ein Blutgerinnsel im Blutstrom eines extrakorporalen Kreislaufes detektiert wird. Das von dem Signalauswertemittel abgegebene Signal wird vorzugsweise in regelmäßigen Zeitabständen (beispielsweise in festen Abständen von wenigen Millisekunden) an die Steuereinheit der Vorrichtung weitergeleitet und dort verarbeitet.

In einer weiteren Ausführungsform kann die erfindungsgemäße Vorrichtung eine Aufnahme aufweisen, in die ein Fluidführungsmittel, eine Kassette oder ein Messkanal einsetzbar ist. Bei dem Fluidführungsmittel handelt es sich vorzugsweise um ein Schlauchsystem oder einen Teil eines Schlauchsystems, beispielsweise um ein Schlauchsystem des extrakorporalen Kreislaufes einer Hämodialysemaschine, einer Herz-Lungen-Maschine oder einer Apherese-Apparatur. Die Kassette kann beispielsweise eine disposable Kassette sein, wie sie in einer Hämodialysemaschine für die Anordnung von Teilen eines extrakorporalen Kreislaufs verwendet werden. Die Vorrichtung weist vorzugsweise einen Messkanal auf, in dem das Ultraschallüberwachungsmittel anordenbar ist.

In einer bevorzugten Ausführungsform kann die erfindungsgemäße Vorrichtung ein Warnmittel aufweisen und/oder mit wenigstens einem Warnmittel in Verbindung stehen. Dies hat den Vorteil, dass mittels des Warnmittels auf die im Fluid oder Fluidstrom erkannten Fremdstrukturen hinweisbar ist. Vorzugsweise ist mit diesem Warnmittel auf Festkörper im Blut eines extrakorporalen Kreislaufes, wie beispielsweise Blutgerinnsel, hinweisbar. Das Warnmittel kann beispielsweise als akustisches (Ton) und/oder optisches (Warnleuchte oder Warnanzeige auf einem Computerbildschirm) Warnmittel sein. Es ist denkbar, dass auf dem Computerbildschirm einer Steuereinheit, die beispielsweise mit einer Hämodialysemaschine, einer Herz-Lungen-Maschine oder einer Apherese-Apparatur in Verbindung steht, gleichzeitig ein Warnhinweis gegeben wird und ein akustisches Warnsignal abgegeben wird.

In einer weiteren bevorzugten Ausführungsform umfasst die erfindungsgemäße Vorrichtung wenigstens ein Schutzmittel oder steht mit wenigstens einem Schutzmittel in Verbindung. Wie oben beschrieben, ist es ein besonderer Vorteil des erfindungsgemäßen Verfahrens, dass Änderungen im Zustand eines Fluidstroms, insbesondere im Gerinnungszustand des Blutes eines extrakorporalen Kreislaufes, instantan detektierbar sind. Die erfindungsgemäße Vorrichtung, die ein Schutzmittel aufweist, hat den Vorteil, dass auf Veränderungen des Zustandes eines Fluidstromes, wie beispielsweise des Gerinnungszustandes von Blut, unmittelbar und ohne Verzögerung reagiert werden kann. Es ist zum Beispiel denkbar, dass unmittelbar nach Detektion wenigstens einer Fremdstruktur, insbesondere wenigstens eines Blutgerinnsels, der Fluidstrom stoppbar ist. Damit kann verhindert werden, dass die sich möglicherweise in einem extrakorporalen Kreislauf, beispielsweise einer Hämodialysemaschine oder einer Herz-Kreislauf-Maschine, bildenden Blutgerinnsel in den mit dem extrakorporalen Kreislauf in Verbindung stehenden Patienten gelangen. Eine weitere Aufgabe des Schutzmittels kann es sein, unmittelbar nach der Detektion einer Fremdstruktur, insbesondere eines Blutgerinnsels, Mittel zur Auflösung dieser Fremdstrukturen in den extrakorporalen Kreislauf zu dosieren. Vorzugsweise können Hemmer der Blutgerinnung in den extrakorporalen Kreislauf zudosiert werden. Damit stehen mit der erfindungsgemäßen Vorrichtung sehr effektive Schutzmittel zur Verfügung, um die Patientensicherheit zu erhöhen und Schaden von den Patienten abzuwenden, die sich einer Behandlung mit beispielsweise einer Hämodialysemaschine oder einer Herz-Kreislauf-Maschine oder einer Apherese-Apparatur unterziehen müssen.

In einer besonders bevorzugten Ausführungsform ist die erfindungsgemäße Vorrichtung Bestandteil einer Blutbehandlungsvorrichtung, insbesondere einer Hämodialysemaschine, einer Herz-Kreislauf-Maschine oder einer Apherese-Apparatur.

Die Erfindung betrifft weiterhin die Verwendung der hierin beschriebenen Vorrichtung zur Durchführung des erfindungsgemäßen Verfahrens. Vorzugsweise betrifft die vorliegende Erfindung auch die Verwendung einer Vorrichtung wie hierin beschrieben, in einer Blutbehandlungsvorrichtung, insbesondere einer Hämodialysemaschine oder einer Herz-Lungen-Maschine oder einer Apherese-Apparatur.

In einer besonders bevorzugten Ausführungsform der vorliegenden Erfindung werden das hierin beschriebene Verfahren und/oder die hierin beschriebene Vorrichtung zur instantanen und/oder individuellen Bestimmung des Verlaufs der intrinsischen und/oder extrinsischen Blutgerinnung in einem extrakorporalen Kreislauf, insbesondere in einer Hämodialysemaschine oder einer Herz-Lungen-Maschine oder einer Apherese-Apparatur, verwendet.

Das erfindungsgemäße Verfahren ist nicht nur auf die Überwachung des Gerinnungszustandes von Blutströmen in extrakorporalen Kreisläufen beschränkt, sondern das erfindungsgemäße Verfahren und/oder die erfindungsgemäße Vorrichtung sind auch in Schnelltestsystemen, wie zum Beispiel in POC (Point-of-Care) Schnellsystemen, im ambulanten und klinischen Einsatz sowie in Labordiagnosegeräte im ambulanten und klinischen Einsatz anwendbar.

Standardmäßig erfolgt die Kontrolle des Gerinnungssystems im Labor, also *in vitro.* Die Ergebnisse liegen je nach Methode zwar nach wenigen Minuten vor, es kommt aber noch die Zeit für Blutentnahme, Transport zum Labor und Kommunikation des Ergebnisses hinzu. Auch modernste mobile Geräte, bei denen der Zeitraum zwischen Blutentnahme und Vorliegen des Ergebnisses bis auf eine Minute verkürzt werden kann, sind auf die invasive Messung beschränkt und nicht zur Überwachung der Gerinnungsparamater, z. B. während einer OP, geeignet.

Die Bestimmung der Gerinnungseigenschaften von Blut erfolgt bisher über die Zugabe gerinnungsaktivierender Substanzen, die an verschiedenen Punkten der Gerinnungskaskade ansetzen, um die Arbeitsweise der verschiedenen Gerinnungsfaktoren bzw. der beiden Wege (extrinsisch, intrinsisch) zu kontrollieren. Die Zeit bis zu einer definierten Gerinnungsstufe, die optisch (mit bloßem Auge oder über Transmissions- bzw. Streumessungen mit einem Sensor) oder mechanisch (z. B. Kugelkoagulometer) gemessen wird, ermöglicht nur eine Aussage über die Wahrscheinlichkeit des Eintretens der Gerinnung im Patienten, nicht aber über den tatsächlichen Gerinnungszustand im Blut.

Das vorliegende Verfahren stellt also eine nicht-invasive Methode zur Bestimmung des Gerinnungszustands von Blut in Echtzeit dar. Im Gegensatz zu den bisher eingesetzten biochemischen Nachweismethoden für Hämostase kann mit Ultraschallrückstreuung keine Aussage über die Gerinnungsfaktoren getroffen werden, dafür aber über den realen Gerinnungszustand, beispielsweise im Vergleich zu Normalblut, der bisher nur aus Erfahrungswerten abgeschätzt werden konnte.

Das erfindungsgemäße Verfahren zur Überwachung eines Fluidzustands kann prinzipiell in allen Bereichen angewendet werden, wo sich der Zustand von Fluiden verändern kann. Dies betrifft beispielweise die Überwachung des Misch- bzw. Entmischungszustandes aller Arten von Suspension, Dispersionen und Emulsionen. Industrielle Anwendungen des erfindungsgemäßen Verfahrens sind beispielsweise die Überwachung des Zustandes von Suspensionen und Dispersionen bei der Herstellung von Schleifmitteln, Farben und Lacken.

Weitere Einzelheiten der Erfindung werden nachfolgend in den Zeichnungen und in einem Ausführungsbeispiel näher erläutert.

Es zeigen:

**Figur 1:** den typischen Aufbau des erfindungsgemäßen Auswertealgorithmus.

**Figur 2:** die Klassifikationsergebnisse der Unterscheidung von Partikelgrößen bei variierenden Volumenkonzentrationen. An den Achsen sind die Größen in $\mu$m gegeben.

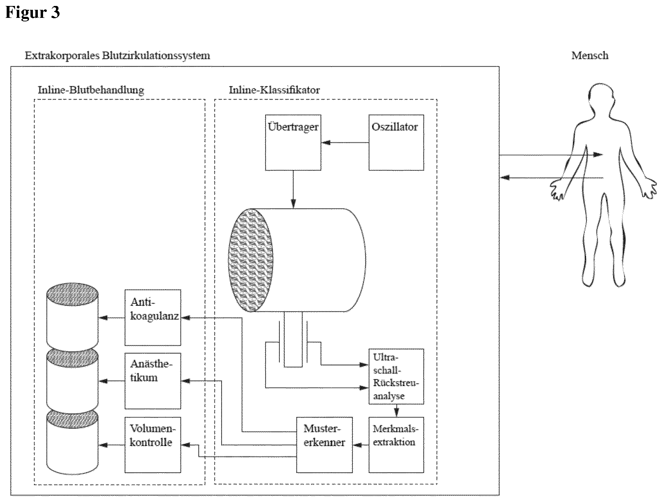

**Figur 3:** den typischen Aufbau eines extrakorporalen Blutkreislaufs mit den entsprechenden Ultraschallüberwachungsmitteln gemäß der vorliegenden Erfindung.

**Beispiel:**

Es sollten Grundlagen zur Streutheorie der Blutzellenaggregation, Auswirkung der Gerinnungskaskade auf die physikalischen Eigenschaften des Blutes und medizinische relevante Parameter erarbeitet werden.

Eine erste Population von Blutersatzflüssigkeiten (Suspensionen) mit leicht variierenden Volumenkonzentrationen bei unterschiedlichen mittleren Teilchengrößen (5, 20 und 40 $\mu$m) wurde genutzt, um einen Auswertealgorithmus zu trainieren. Daran wurde eine zweite Population mit gleichen Volumenkonzentrationen und Teilchengrößen verwendet, um die Güte des Auswertealgorithmus zu testen.

1. Technischer Aufbau

Für den experimentellen Nachweis wurde ein Ultraschallgerät verwendet, welches ein hohes Signal-Rausch-Verhältnis besitzt. Über das Einbringen eines Ultraschallbursts (mehrere Schwingungsperioden) wurde das Gerät für die Doppleranalyse optimiert. Zusätzlich wurde eine Auswertesoftware implementiert, die eine hohe Abtastrate der Pulswiederholfolge erlaubt. Diese ist notwendig, um für die elastographische Auswertung Shannon's Abtasttheorem zu genügen. Die Klassifikation wurde mit Matlab (The MathWorks Inc.) vorgenommen.

Der mechanische Aufbau wurde auf einer Vibrationsliege mit zwei elektromechanischen Wandlern vorgenommen. Auf den zwei Wandlern stand eine Halterung für die Küvette. Durch gegenphasige Ansteuerung der Wandler wurde die Suspension in Schwingung versetzt. Der Aufbau umfasste einen Auswertecomputer, Netzteile zur Speisung von Rührer und dem Ultraschallinterface sowie eine Küvette, die auf einer Küvettenhalterung bzw. den Wandlern, montiert wurde.

Durch die planparallelen Wände konnte ein komplexes Netz an Moden erzeugt werden, welche mit den viskosen Eigenschaften einer Suspension in Wechselwirkung stehen. Für die Dopplermessungen wurde ein Rührer in die Suspension gesenkt und bei verschiedenen Rührgeschwindigkeiten wurden Ultraschallmessungen vorgenommen. Für reine Rückstreumessungen wurde die Suspension aufgerührt und bei einer sehr geringen Rotationsgeschwindigkeit des Rührers gemessen.

### 2. Herstellung einer blutähnlichen Flüssigkeit

In vielen Bereichen der medizinischen Forschung wird menschliches Blut nicht direkt eingesetzt, da es sich u. a. schlecht lagern lässt. Weil oft nur wenige, bestimmte Eigenschaften verwendet werden, die sich nur in diesen Eigenschaften wie echtes Blut verhalten. Neben Tierblut kommen dabei auch teil- und vollsynthetische Flüssigkeiten zum Einsatz, sogenannte Blutersatzflüssigkeiten (BEF, bzw. im Englischen blood mimicking fluids - BMF). Für Untersuchungen zur Detektion von Blutgerinnung (Hämostase) mittels Ultraschallrückstreuung kommen in diesem Beispiel geeignete BEF zum Einsatz.

BEF Voruntersuchungen zur Rückstreu-Methode wurden an verschiedenen BEF durchgeführt, an die folgende Anforderungen gestellt wurden:

- verschiedene Partikelgrößen,
- Suspensionen, d. h. trennbare Partikel-Lösungsmittel-Gemische,
- chemische und physikalische Stabilität der BEF.

Unter Verwendung verschiedener Stoffe wurden BEF hergestellt. Die Abhängigkeit der BEF-Eigenschaften von den Stoffeigenschaften wurde untersucht.

Material: Es wurden zwei verschiedene Stoffe für die Herstellung von BEF verwendet: ORGASOL® (Arkema Inc.) und megaCRYL® (megadental GmbH).

**Tabelle 3:** Eigenschaften der verwendeten Stoffe

|  | megaCRYL® | ORGASOL® |
|---|---|---|
| Material | PMMA | PA 12 |
| Durchmesser | 10 - 60 $\mu$m | 5 $\mu$m; 20 $\mu$m; 40 $\mu$m |
| Dichte | 1,16 g/cm$^3$ | 1,03 g/cm$^3$ |
| Ursp. Verwendungszweck | Kaltpolymerisat Dentallabor | Additiv Farbenindustrie |

PMMA     *Polymethylmethacrylat*
PA12     *Polyamid 12*

Beide Stoffe zeigten unterschiedliches Verhalten beim Anrühren und der Lagerbarkeit der Suspensionen, wie in Tabelle 4 dargestellt.

**Tabelle 4:** Eigenschaften der hergestellten BEF

|  | megaCRYL® | ORGASOL® | ORGASOL® | ORGASOL® |
|---|---|---|---|---|
| Streukörperdurchmesser | 10 - 60 $\mu$m | 5 $\mu$m | 20 $\mu$m | 40 $\mu$m |
| Dispersität | poly dispers | monodispers | monodispers | monodispers |
| Suspendierbarkeit in Wasser | sehr gut | schlecht | gut | gut |
| Suspendierbarkeit in Wasser | sehr gut | schlecht | gut | gut |
| Viskosität durch Konzentration beeinflusst | ja | nein | nein | nein |
| Resuspendierbarkeit | mäßig | mäßig | gut | gut |
| ehem. Stabilität | Polymeration | 1) | 1) | 1) |
| phys. Stabilität | 1) | 1) | 1) | 1) |
| biol. Stabilität | 2) | 2) | 2) | 2) |
| höchste hergestellte Konzentration | 33 M. % | 25 M. % | 18 M. % | 18 M. % |

*1) bisher keine Veränderung beobachtet worden, d. h., die Suspensionen sind über Monate unverändert geblieben.*
*2) die Suspensionen enthalten keine biologischen Komponenten, und*
*3) mit bisher verwendeten Additiven maximal mögliche Konzentration.*

*Viskositätsmessung*

Bei den megaCRYL®-Suspensionen konnte eine Abhängigkeit der Viskosität von der Konzentration ermittelt werden. Die ORGASOL® -Suspensionen haben eine ähnliche Viskosität wie Wasser.

*Datenbasis*

Für einen Nachweis der Klassifikationsmethode wurden die Suspensionen in Tabelle 5 erstellt. Wie in der späteren Anwendung an der Dialysemaschine, soll trotz einer variierenden Volumenkonzentration (Hämatokrit) die sichere Erkennung des Teilchendurchmessers von einzelnen Blutzellen und ihren Mikroaggregaten möglich sein.

**Tabelle 5:** Datenbasis für Klassifikationsnachweis von Suspensionen, die sich neben der Teilchenanzahl über den Teilchendurchmesser unterscheiden.

| Suspension (Set/Durchmesser) | Volumenprozent |
| --- | --- |
| 1/5 | 10,1 ± 0,23 |
| 1/20 | 9,8 ± 0,24 |
| 1/40 | 9,3 ± 0,25 |
| 2/5 | 11,7 ± 0,24 |
| 2/20 | 11,7 ± 0,24 |
| 2/40 | 11,7 ± 0,24 |
| 3/5 | 13,8 ± 0,25 |
| 3/20 | 13,8 ± 0,25 |
| 3/40 | 13,6 ± 0,24 |
| 4/5 | 14,4 ± 0,25 |
| 4/20 | 14,6 ± 0,25 |
| 4/40 | 14,5 ± 0,25 |
| 5/5 | 15,7 ± 0,25 |
| 5/20 | 15,8 ± 0,26 |
| 5/40 | 15,8 ± 0,26 |
| 6/5 | 17,6 ± 0,26 |
| 6 / 20 | 1 7,8 ± 0,26 |
| 6/40 | 17,5 ± 0,26 |

3. Durchführung der Messungen

Die Messungen wurden unter Verwendung der Software ft2232 (GAMPT) mit folgenden Einstellungen vorgenommen: prt 1999 (Plus-Wiederholrate), data num. 2048 (Anzahl Datenpunkte des A-Scans), delay 5 (Beginn der Aufzeichnung der Datenpunkte), gain 50 (analoge Verstärkung in dB re 1V), transmitter 30 (analoge Verstärkung Übertrager dB re 1 V), filter 1 100 (keine Filterung), filter 2 100 (keine Filterung), spl 4000 (Aufnahme 4000 A-Scan-Linien), fs 50 MHz (Abtastfrequenz), Burst f 3,125 Mhz (Signalfrequenz), TGC max. (digitale Verstärkung voll) im Messbereich unter Verwendung eines Panametrics-Wandlers mit einer Mittenfrequenz von 3,5 MHz.

Aus den Messungen der Sets 4 bis 6 wurden an zeitlich verschiedenen Proben die Merkmale Rückstreuung, Doppler und Elastographie über eine Aufnahmelänge von jeweils 0,6 s bestimmt. Dergestalt lagen für das Training 9 Suspensionen x 7 Anregungsformen (Rührgeschwindigkeit, usw.) x 6 verschiedene Zeitproben vor. Die Messungen und Merkmalsentnahmen der 7 Anregungsformen wurden in einem Vektor angeordnet, was schließlich in 54 Proben resultierte. Die gleiche Anzahl wurde für die Evaluation des Auswertealgorithmus benutzt. Beide Populationen sind voneinander zeitlich unabhängig. Die Auswertung von Rückstreuung, Doppler und Elastographie erfolgte für jede Messung, unabhängig von der Anregungsform. Der Tiefenbereich wurde auf 300 Datenpunkte im Bereich der Rückstreuung beschränkt, um Einflüsse durch unterschiedliche Füllstände in der Küvette zu vermeiden. Jede Probe resultierte in einen Vektor von 7 Anregungsarten x jeweils 1068 Merkmalen. Alle Merkmale wurden nach ihrer Berechnung logarithmiert (Basis e).

Die sieben Anregungsformen umfassten eine Speisung des Rührers mit 0,7; 1; 1,5 und 2 V (Hewlett Packard E3611A, bei voller Stromstärke) sowie die harmonische Anregung mit 5; 8 und 15 Hz (Verstärker Typ tamp TSA 1400 bei halber Leistung, PC Soundkarte Typ Realtek Audio High Definition Soundkarte volle Verstärkung, Matlab bei einem Amplitudenfaktor von 0,5) bei angeschaltetem Rührer. Vor jeder Messung wurde die Suspension kurz aufgerührt, um Sedimentation zu vermeiden. Die Rührgeschwindigkeit bei 0,7 V entspricht pro A-Scan einem stationären Zustand der Suspension, was als Kriterium für die hier ausgeführte Rückstreuanalyse gefordert wurde.

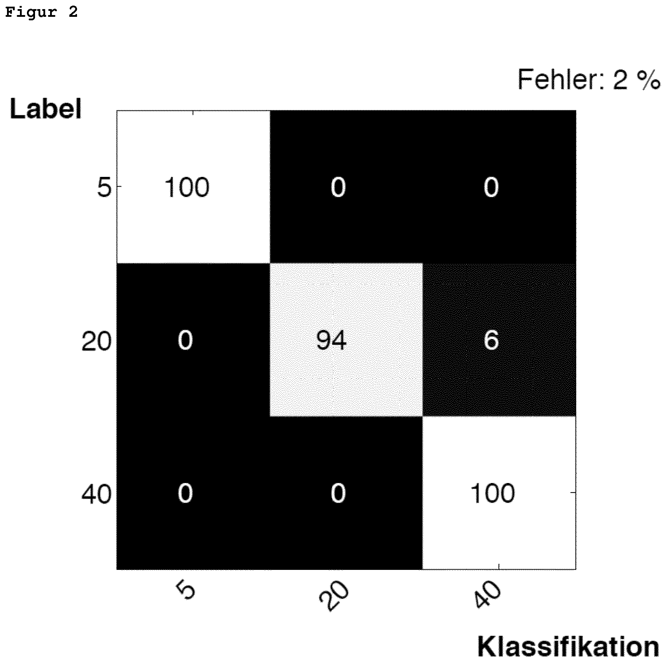

Die Evaluationsergebnisse des trainierten Auswertealgorithmus sind in Abbildung 2 in einer sogenannten Konfusionsmatrix dargestellt. Die Ordinate gibt die tatsächlichen Label an, hier den Durchmesser. Die Abszisse bezeichnet die Klassifikationsergebnisse. Die Resultate sind in Prozent dargestellt. Die perfekte Klassifikation würde zu einer Hauptdiagonalen mit Werten von jeweils 100 % führen. Tatsächlich wird eine sehr hohe Klassifikationsgüte mit einem Fehler von 2 % erreicht.

Die Ergebnisse der beschriebenen Messung erbringen den Nachweis des vorgestellten Ansatzes der multikriteriellen Untersuchung von Fluiden zur Bestimmung von Partikelgrößenunterschieden bei variierenden Volumenkonzentrationen weit unter der Auflösung des Ultraschallsignals (die Wellenlänge des verwendeten Ultraschallsignals in Wasser betrug 480 $\mu$m).

## Patentansprüche

1. Verfahren zur Ermittlung und/oder Überwachung des Zustandes eines extrakorporalen Fluides oder eines extrakorporalen Fluidstroms, wobei das Fluid mittels Ultraschall überwacht wird, **dadurch gekennzeichnet, dass** das Fluid Blut ist, welches in einem extrakorporalen Kreislauf einer extrakorporalen Blutbehandlungsmaschine vorliegt und das extrakorporale Fluid oder der extrakorporale Fluidstrom zeitharmonisch mechanisch und/oder transient mechanisch angeregt wird und durch das Ultraschall-Rückstreusignal instantan und gleichzeitig die Dopplerfrequenzverschiebung und die Viskosität ermittelt werden, die mittels der Ultraschallüberwachung ermittelten Merkmale des Zustandes des extrakoporalen Fluids mit einer multikriteriellen Ultraschallanalyse, insbesondere einem Auswertealgorithmus, verarbeitet werden, wodurch eine Änderung der Verteilung von in dem Fluid enthaltenen Teilchen und Änderungen der Viskosität des Fluides mittels Ultraschall gemessen werden und der Fluidzustand zuvor definierten Zuständen zugewiesen wird.

2. Verfahren nach Anspruch 1 zur Ermittlung und/oder Überwachung des Zustandes von Blut eines extrakorporalen Blutstroms, wobei das Blut mittels Ultraschall überwacht wird, **dadurch gekennzeichnet, dass** der extrakorporale Blutstrom zeitharmonisch mechanisch und/oder transient mechanisch angeregt wird und das Ultraschall-Rückstreusignal hinsichtlich der Dopplerfrequenzverschiebung und der Elastographie analysiert wird, die mittels der Ultraschallüberwachung ermittelten Merkmale des Zustandes des extrakorporalen Blut mit einer multikriteriellen Ultraschallanalyse, insbesondere einem Auswertealgorithmus, verarbeitet werden, wodurch eine Änderung der Verteilung von Bestandteilen im Blut und Änderungen der Viskosität des Bluts mittels Ultraschall gemessen werden und der Blutzustand zuvor definierten Zuständen, insbesondere Gerinnungszuständen, zugewiesen wird.

3. Verfahren nach einem der Ansprüche 1 oder 2, **dadurch gekennzeichnet, dass** wenigstens eine erste Fremdstruktur, insbesondere ein Festkörper wie ein Blutgerinnsel, im extrakorporalen Fluid erkennbar und/oder von wenigstens einer zweiten festen/flüssigen/gasförmigen Fremdstruktur, z. B. Luftblasen, über die Rückstreuamplitude unterschieden wird.

4. Verfahren nach mindestens einem der vorherigen Ansprüche, **dadurch gekennzeichnet, dass** mittels der Ultraschall-Rückstreuung eine Vielzahl von Merkmalen des Blutzustandes augenblicklich ermittelt werden.

5. Verfahren nach mindestens einem der vorherigen Ansprüche, **dadurch gekennzeichnet, dass** der Auswertealgorithmus die nachfolgenden Signalverarbeitungsstufen umfasst:

   a. Empfangen eines Ultraschallsignals oder mehrerer Ultraschallsignale,
   b. Merkmalsextraktion aus dem (den) Ultraschallsignal(en),
   c. Mustererkennung,
   d. Nachverarbeitung, und
   e. Zuordnung einer Punktzahl, wobei die Punktzahl die Güte (Wahrscheinlichkeit) für ein gegebenes Klassifikationsresultat definiert.

6. Verfahren nach Anspruch 5, **dadurch gekennzeichnet, dass** auf Grundlage eines Maschinenlernalgorithmus alle Signalverarbeitungsstufen offline optimiert werden.

7. Verfahren nach mindestens einem der vorherigen Ansprüche, **dadurch gekennzeichnet, dass** der individuelle Zustand des extrakorporalen Fluides, insbesondere Blutzustands, von dem Auswertealgorithmus zusätzlich durch eine fortlaufende Optimierung online gelernt wird.

8. Verfahren nach mindestens einem der vorherigen Ansprüche, **dadurch gekennzeichnet, dass** mittels Ultraschall-Rückstreuung physikalische Parameter, die durch die fortschreitende Blutgerinnung verändert werden können, gemessen werden..

9. Verfahren nach mindestens einem der vorherigen Ansprüche, **dadurch gekennzeichnet, dass** mittels Ultraschall-Rückstreuung physikalische Parameter zur Detektion beginnender Blutgerinnung gemessen werden.

10. Verfahren nach Anspruch 8, **dadurch gekennzeichnet dass** die physikalischen Parameter ausgewählt sind aus Schallgeschwindigkeit, Durchmesser des Streukörpers, die Standardabweichung der Rückstreuung, die maximale Abweichung der Rückstreuung, das Maß der Regelmäßigkeit der Anordnung der Streukörper, die Turbulenz und die Geschwindigkeitsverteilung der Streukörper, der Druck im Schlauchsystem.

11. Verfahren nach mindestens einem der vorherigen Ansprüche, **dadurch gekennzeichnet, dass** die Aggregation von Thrombozyten und/oder Erythrozyten gemessen wird.

12. Verfahren nach mindestens einem der vorherigen Ansprüche, **dadurch gekennzeichnet, dass** Fehler in einem extrakorporalen Fluidführungsmittel, vorzugsweise einem Schlauchsystem einer Vorrichtung, detektiert werden.

13. Vorrichtung zur Durchführung des Verfahrens nach mindestens einem der vorherigen Ansprüche, umfassend wenigstens ein Ultraschallüberwachungsmittel und wenigstens ein Signalauswertemittel, **dadurch gekennzeichnet, dass** die Vorrichtung weiterhin ein Mittel zur zeitharmonischen mechanischen Anregung des Fluids, welches den extrakorporalen Fluidstrom, insbesondere den extrakorporalen Blutstrom, zeitharmonisch mechanisch und/oder transient mechanisch anregt; und einen Auswertealgorithums, welcher die mittels Ultraschallüberwachung ermittelten Merkmale des Zustands des extrakorporalen Fluids, insbesondere des Zustands von extrakorporalem Blut verarbeitet und den Zustand des extrakorporalen Fluids, insbesondere des extrakorporalen Bluts, zuvor definierten Zuständen, insbesondere Gerinnungszuständen zuordnet, umfasst; wobei die Vorrichtung dazu eingerichtet ist durch das Ultraschall-Rückstreusignal instantan und gleichzeitig die Dopplerfrequenzverschiebung und die Viskosität zu ermitteln; und das Fluid Blut ist, welches in einem extrakorporalen Kreislauf einer extrakorporalen Blutbehandlungsmaschine vorliegt.

14. Vorrichtung nach Anspruch 13, **dadurch gekennzeichnet, dass** mittels des Signalauswertemittels durch Auswertung der Rückstreuamplitude wenigstens eine erste Fremdstruktur, insbesondere ein oder mehrere Festkörper, insbesondere Blutgerinnsel, im extrakorporalen Fluid erkennbar und/oder von wenigstens einer zweiten Fremdstruktur unterscheidbar sind.

15. Vorrichtung nach Anspruch 13 oder 14, **dadurch gekennzeichnet, dass** mittels des Signalauswertemittels Änderungen der Verteilung von in dem extrakorporalen Fluid enthaltenen Teilchen, insbesondere Blutbestandteilen, und Änderungen der Viskosität des extrakorporalen Fluides, insbesondere extrakorporalen Bluts als signalauslösendes Ereignis registriert wird und ein Signal an eine Steuereinheit der Vorrichtung abgegeben wird.

16. Vorrichtung nach mindestens einem der Ansprüche 13-15, **dadurch gekennzeichnet, dass** die Vorrichtung eine Aufnahme aufweist, in die ein Fluidführungsmittel, vorzugsweise ein Schlauchsystem oder ein Teil eines Schlauchsystems oder eine Kassette, insbesondere eine Disposablekassette, oder ein Messkanal, einsetzbar ist und/oder dass die Vorrichtung einen Messkanal aufweist.

17. Vorrichtung nach mindestens einem der Ansprüche 13-16, **dadurch gekennzeichnet, dass** die Vorrichtung wenigstens ein Warnmittel aufweist und/oder mit wenigstens einem Warnmittel in Verbindung steht, wobei mittels des Warnmittels auf Änderungen der Verteilung von in dem extrakorporalen Fluid enthaltenen Teilchen, insbesondere Blutbestandteilen, und Änderungen der Viskosität des extrakorporalen Fluides, insbesondere extrakorporalen Bluts, hinweisbar ist.

**18.** Vorrichtung nach mindestens einem der Ansprüche 13-17, **dadurch gekennzeichnet, dass** die Vorrichtung wenigstens ein Schutzmittel aufweist und/oder mit wenigstens einem Schutzmittel in Verbindung steht, wobei mittels des Schutzmittels vorzugsweise der extrakorporale Fluidstrom stoppbar ist und/oder mindestens ein Mittel zur Korrektur des Zustands des extrakorporalen Fluides, insbesondere des Gerinnungszustands von extrakorporalem Blut, wie Hemmer der Blutgerinnung, zu dem Fluidstrom, insbesondere dem extrakorporalen Kreislauf, zudosierbar sind.

**19.** Verwendung des Verfahrens nach mindestens einem der Ansprüche 1-12 in Schnelltestsystemen, wie zum Beispiel in POC (Point-of-Care) Schnellsystemen; oder in Labordiagnosegeräten, im ambulanten und/oder klinischen Einsatz, wobei das Verfahren extrakorporal durchgeführt wird.

**Claims**

**1.** A method for determining and/or monitoring the state of an extracorporeal fluid or an extracorporeal fluid flow, wherein the fluid is monitored by means of ultrasound, **characterised in that** the fluid is blood which is present in an extracorporeal circuit of an extracorporeal blood treatment machine and the extracorporeal fluid or the extracorporeal fluid flow is excited time-harmonically mechanically and/or transiently mechanically and the Doppler frequency shift and the viscosity are determined instantaneously and simultaneously by the ultrasound backscatter signal, the characteristics of the state of the extracoporal fluid determined by means of the ultrasonic monitoring are processed with a multi-criteria ultrasonic analysis, in particular an evaluation algorithm, whereby a change in the distribution of particles contained in the fluid and changes in the viscosity of the fluid are measured by means of ultrasound and the fluid state is assigned to previously defined states.

**2.** A method according to claim 1 for determining and/or monitoring the state of blood of an extracorporeal blood flow, wherein the blood is monitored by means of ultrasound, **characterized in that** the extracorporeal blood flow is time-harmonically mechanically and/or transiently mechanically excited and the ultrasound backscatter signal is analysed with respect to Doppler frequency shift and elastography, the characteristics of the state of the extracorporeal blood determined by means of the ultrasound monitoring are processed with a multi-criteria ultrasound analysis, in particular an evaluation algorithm, whereby a change in the distribution of constituents in the blood and changes in the viscosity of the blood are measured by means of ultrasound and the blood state is assigned to previously defined states, in particular coagulation states.

**3.** Method according to one of claims 1 or 2, **characterized in that** at least one first foreign structure, in particular a solid body such as a blood clot, is detectable in the extracorporeal fluid and/or distinguished from at least one second solid/liquid/gaseous foreign structure, e.g. air bubbles, via the backscatter amplitude.

**4.** Method according to at least one of the previous claims, **characterized in that** a plurality of features of the blood state are instantaneously determined by means of the ultrasonic backscatter.

**5.** Method according to at least one of the previous claims, **characterized in that** the evaluation algorithm comprises the following signal processing stages:

    a. Receiving one or more ultrasound signals,
    b. Feature extraction from the ultrasonic signal(s),
    c. Pattern recognition,
    d. Post-processing, and
    e. Assigning a score, wherein the score defines the quality (probability) for a given classification result.

**6.** Method according to claim 5, **characterized in that** all signal processing stages are optimized offline on the basis of a machine learning algorithm.

**7.** Method according to at least one of the previous claims, **characterized in that** the individual state of the extracorporeal fluid, in particular blood state, is additionally learned online by the evaluation algorithm by continuous optimization.

**8.** Method according to at least one of the previous claims, **characterized in that** physical parameters which can be changed by progressive blood coagulation are measured by means of ultrasound backscattering.

9. Method according to at least one of the preceding claims, **characterized in that** physical parameters for detecting incipient blood coagulation are measured by means of ultrasound backscatter.

10. Method according to claim 8, **characterized in that** the physical parameters are selected from sound velocity, diameter of the scattering body, the standard deviation of the backscattering, the maximum deviation of the backscattering, the degree of regularity of the arrangement of the scattering bodies, the turbulence and the velocity distribution of the scattering bodies, the pressure in the tube system.

11. Method according to at least one of the previous claims, **characterized in that** the aggregation of platelets and/or erythrocytes is measured.

12. Method according to at least one of the previous claims, **characterized in that** defects in an extracorporeal fluid conducting means, preferably a tubing system of a device, are detected.

13. Device for executing the method according to at least one of the preceding claims, comprising at least one ultrasound monitoring means and at least one signal evaluation means, **characterized in that** the apparatus further comprises a means for time-harmonic mechanical excitation of the fluid, which means excites the extracorporeal fluid flow, in particular the extracorporeal blood flow, time-harmonically mechanically and/or transiently mechanically; and an evaluation algorithm which processes the features of the state of the extracorporeal fluid, in particular the state of extracorporeal blood, determined by means of ultrasound monitoring and assigns the state of the extracorporeal fluid, in particular the extracorporeal blood, to previously defined states, in particular coagulation states;
wherein the device is adapted to instantaneously and simultaneously determine the Doppler frequency shift and the viscosity by the ultrasonic backscatter signal;
and the fluid is blood present in an extracorporeal circuit of an extracorporeal blood treatment machine.

14. Device according to claim 13, **characterized in that** by means of the signal evaluation means at least one first foreign structure, in particular one or more solid bodies, in particular blood clots, can be detected in the extracorporeal fluid and/or can be distinguished from at least one second foreign structure by evaluating the backscatter amplitude.

15. Device according to claim 13 or 14, **characterized in that** changes in the distribution of particles contained in the extracorporeal fluid, in particular blood components, and changes in the viscosity of the extracorporeal fluid, in particular extracorporeal blood, are detected as a signal-triggering event by means of the signal evaluation means and a signal is emitted to a control unit of the device.

16. Device according to at least one of claims 13-15, **characterized in that** the device has a receptacle into which a fluid guiding means, preferably a hose system or part of a hose system or a cassette, in particular a disposable cassette, or a measuring channel, can be inserted and/or that the device has a measuring channel.

17. Device according to at least one of claims 13-16, **characterized in that** the device has at least one warning means and/or is connected to at least one warning means, it being possible to use the warning means to indicate changes in the distribution of particles contained in the extracorporeal fluid, in particular blood constituents, and changes in the viscosity of the extracorporeal fluid, in particular extracorporeal blood.

18. Device according to at least one of claims 13-17, **characterized in that** the device comprises at least one protective means and/or is connected to at least one protective means, wherein by means of the protective means preferably the extracorporeal fluid flow can be stopped and/or at least one means for correcting the state of the extracorporeal fluid, in particular the coagulation state of extracorporeal blood, such as inhibitors of blood coagulation, can be metered to the fluid flow, in particular the extracorporeal circuit.

19. Use of the method according to at least one of claims 1-12 in rapid test systems, such as in POC (point-of-care) rapid systems; or in laboratory diagnostic devices, in ambulatory and/or clinical use, wherein the method is performed extracorporeally.

**Revendications**

1. Procédé pour la détection et/ou la surveillance de l'état d'un fluide extracorporel ou d'un courant de fluide extracorporel, le fluide étant surveillé au moyen d'ultrasons, **caractérisé en ce que** le fluide est du sang, qui se trouve dans un circuit extracorporel d'une machine de traitement de sang extracorporelle, et le fluide extracorporel ou le courant de fluide extracorporel est stimulé mécaniquement de manière harmonieuse dans le temps et/ou mécaniquement de manière transitoire, et le décalage de fréquence Doppler et la viscosité sont déterminés instantanément et simultanément par le signal de rétrodiffusion d'ultrasons, les caractéristiques de l'état du fluide extracorporel déterminées au moyen de la surveillance par ultrasons sont traitées avec une analyse d'ultrasons multicritère, notamment un algorithme d'évaluation, par laquelle une modification de la distribution de particules contenues dans le fluide et des modifications de la viscosité du fluide sont mesurées au moyen d'ultrasons, et l'état du fluide est attribué à des états définis auparavant.

2. Procédé selon la revendication 1 pour la détection et/ou la surveillance de l'état de sang d'un courant de sang extracorporel, le sang étant surveillé au moyen d'ultrasons, **caractérisé en ce que** le courant de sang extracorporel est stimulé mécaniquement de manière harmonieuse dans le temps et/ou mécaniquement de manière transitoire et le signal de rétrodiffusion d'ultrasons est analysé au regard du décalage de fréquence Doppler et de l'élastographie, les caractéristiques de l'état du sang extracorporel déterminées au moyen de la surveillance par ultrasons sont traitées avec une analyse d'ultrasons multicritère, notamment un algorithme d'évaluation, par laquelle une modification de la distribution de constituants dans le sang et des modifications de la viscosité du sang sont mesurées au moyen d'ultrasons, et l'état du sang est attribué à des états définis auparavant, notamment des états de coagulation.

3. Procédé selon l'une quelconque des revendications 1 ou 2, **caractérisé en ce qu'**au moins une première structure étrangère, notamment un corps solide tel qu'un caillot sanguin, est détectable dans le fluide extracorporel et/ou est différenciée d'au moins une deuxième structure étrangère solide/liquide/gazeuse, p. ex. des bulles d'air, par l'intermédiaire de l'amplitude de rétrodiffusion.

4. Procédé selon au moins l'une quelconque des revendications précédentes, **caractérisé en ce qu'**une pluralité de caractéristiques de l'état du sang sont déterminées immédiatement au moyen de la rétrodiffusion d'ultrasons.

5. Procédé selon au moins l'une quelconque des revendications précédentes, **caractérisé en ce que** l'algorithme d'évaluation comprend les étapes de traitement de signal suivantes :

   a. la réception d'un signal ultrasonore ou de plusieurs signaux ultrasonores,
   b. l'extraction de caractéristiques à partir du ou des signaux ultrasonores,
   c. la reconnaissance de motifs,
   d. le post-traitement, et
   e. l'attribution d'un nombre de points, le nombre de points définissant la qualité (probabilité) pour un résultat de classification donné.

6. Procédé selon la revendication 5, **caractérisé en ce que** toutes les étapes de traitement de signal sont optimisées hors ligne sur la base d'un algorithme d'apprentissage automatique.

7. Procédé selon au moins l'une quelconque des revendications précédentes, **caractérisé en ce que** l'état individuel du fluide extracorporel, notamment l'état du sang, est en outre appris en ligne par une optimisation continue par l'algorithme d'évaluation.

8. Procédé selon au moins l'une quelconque des revendications précédentes, **caractérisé en ce que** des paramètres physiques, qui peuvent être modifiés par la progression de la coagulation sanguine, sont mesurés au moyen de la rétrodiffusion d'ultrasons.

9. Procédé selon au moins l'une quelconque des revendications précédentes, **caractérisé en ce que** des paramètres physiques pour la détection du début de la coagulation sanguine sont mesurés au moyen de la rétrodiffusion d'ultrasons.

10. Procédé selon la revendication 8, **caractérisé en ce que** les paramètres physiques sont choisis parmi la vitesse du son, le diamètre du corps de diffusion, l'écart-type de la rétrodiffusion, l'écart maximal de la rétrodiffusion, la mesure de la régularité de l'agencement des corps de diffusion, la turbulence et la distribution de vitesses des corps de diffusion, la pression dans le système de tuyaux.

11. Procédé selon au moins l'une quelconque des revendications précédentes, **caractérisé en ce que** l'agrégation de thrombocytes et/ou d'érythrocytes est mesurée.

**12.** Procédé selon au moins l'une quelconque des revendications précédentes, **caractérisé en ce que** des défauts dans un moyen de conduction de fluide extracorporel, de préférence un système de tuyaux d'un dispositif, sont détectés.

**13.** Dispositif pour la réalisation du procédé selon au moins l'une quelconque des revendications précédentes, comprenant au moins un moyen de surveillance par ultrasons et au moins un moyen d'évaluation de signal, **caractérisé en ce que** le dispositif comprend par ailleurs un moyen pour l'excitation mécanique harmonieuse dans le temps du fluide, qui excite mécaniquement de manière harmonieuse dans le temps et/ou mécaniquement de manière transitoire le courant de fluide extracorporel, notamment le courant de sang extracorporel ; et un algorithme d'évaluation, qui traite les caractéristiques de l'état du fluide extracorporel, notamment de l'état de sang extracorporel, déterminées au moyen de la surveillance par ultrasons, et attribue l'état du fluide extracorporel, notamment du sang extracorporel, à des états définis auparavant, notamment des états de coagulation ;
le dispositif étant conçu pour déterminer instantanément et simultanément le décalage de fréquence Doppler et la viscosité par le signal de rétrodiffusion d'ultrasons ;
et le fluide étant du sang, qui se trouve dans un circuit extracorporel d'une machine de traitement de sang extracorporelle.

**14.** Dispositif selon la revendication 13, **caractérisé en ce qu'**au moyen du moyen d'évaluation de signal, par évaluation de l'amplitude de rétrodiffusion, au moins une première structure étrangère, notamment un ou plusieurs corps solides, notamment un caillot sanguin, est détectable dans le fluide extracorporel et/ou différenciable d'au moins une deuxième structure étrangère.

**15.** Dispositif selon la revendication 13 ou 14, **caractérisé en ce qu'**au moyen du moyen d'évaluation de signal, des modifications de la distribution de particules contenues dans le fluide extracorporel, notamment de constituants sanguins, et des modifications de la viscosité du fluide extracorporel, notamment du sang extracorporel, sont enregistrées en tant qu'événement déclenchant un signal et un signal est émis vers l'unité de commande du dispositif.

**16.** Dispositif selon au moins l'une quelconque des revendications 13 à 15, **caractérisé en ce que** le dispositif comprend un logement, dans lequel un moyen de conduction de fluide, de préférence un système de tuyaux ou une partie d'un système de tuyaux ou une cassette, notamment une cassette jetable, ou un canal de mesure, est insérable et/ou **en ce que** le dispositif comprend un canal de mesure.

**17.** Dispositif selon au moins l'une quelconque des revendications 13 à 16, **caractérisé en ce que** le dispositif comprend au moins un moyen d'avertissement et/ou est relié avec au moins un moyen d'avertissement, des modifications de la distribution de particules contenues dans le fluide extracorporel, notamment de constituants sanguins, et des modifications de la viscosité du fluide extracorporel, notamment du sang extracorporel, pouvant être signalées au moyen du moyen d'avertissement.

**18.** Dispositif selon au moins l'une quelconque des revendications 13 à 17, **caractérisé en ce que** le dispositif comprend au moins un moyen de protection et/ou est relié avec au moyen un moyen de protection, le courant de fluide extracorporel pouvant de préférence être stoppé et/ou au moins un moyen pour la correction de l'état du fluide extracorporel, notamment de l'état de coagulation de sang extracorporel, tel qu'un inhibiteur de la coagulation sanguine, pouvant être dosé dans le courant de fluide, notamment le circuit extracorporel au moyen du moyen de protection.

**19.** Utilisation du procédé selon au moins l'une quelconque des revendications 1 à 12 dans des systèmes de test rapide, tels que par exemple dans des systèmes rapides POC (Point of Care) ; ou dans des appareils de diagnostic de laboratoire, dans l'utilisation ambulatoire et/ou clinique, le procédé étant réalisé de manière extracorporelle.

**Figur 1**

```
          ┌─────────────────┐
          │  Ultraschall-   │
          │     signal      │
          └────────┬────────┘
                   │
                   ▼
          ┌─────────────────┐
          │   Merkmals-     │
          │   extraktion    │
          └────────┬────────┘
                   │
                   ▼
          ┌─────────────────┐
          │    Muster-      │
          │    erkenner     │
          └────────┬────────┘
                   │
                   ▼
          ┌─────────────────┐
          │     Nach-       │
          │  verarbeitung   │
          └────────┬────────┘
                   │
                   ▼
          ┌─────────────────┐
          │   Punktzahl     │
          └─────────────────┘
```

Figur 2

Fehler: 2 %

**Figur 3**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102010034553
- DE 2911258 B1
- US 5928180 A
- DE 10311408 B3
- US 2004065143 A

### In der Beschreibung aufgeführte Nicht-Patentliteratur

- **KALMAN, R. E.** A new approach to linerar filtering and prediction problems. *Transaction of the ASME, Journal of Basic Engineering,* 1960, 35-45
- Using discriminant analysis for multi-class classification. **LI et al.** Proceedings of the Third IEEE International Conference on Data Mining. IEEE Computer Society, 2003, 589